(19) Europäisches Patentamt
European Patent Office
Office européen des brevets

(11) EP 3 903 783 B1

(12) # EUROPEAN PATENT SPECIFICATION

(45) Date of publication and mention of the grant of the patent:
**05.03.2025 Bulletin 2025/10**

(21) Application number: **19905022.0**

(22) Date of filing: **24.12.2019**

(51) International Patent Classification (IPC):
***A61K 31/4965*** (2006.01) ***A61K 31/519*** (2006.01)
***A61K 31/52*** (2006.01) ***A61K 31/495*** (2006.01)
***A61K 31/522*** (2006.01) ***A61K 31/7056*** (2006.01)
***A61K 45/06*** (2006.01) ***A61P 31/14*** (2006.01)
***A61P 43/00*** (2006.01)

(52) Cooperative Patent Classification (CPC):
(C-Sets available)
**A61K 45/06; A61K 31/495; A61K 31/4965; A61K 31/519; A61K 31/52; A61K 31/522; A61K 31/7056; A61P 31/14; A61P 43/00;**
Y02A 50/30; Y02P 20/55 (Cont.)

(86) International application number:
**PCT/JP2019/050578**

(87) International publication number:
**WO 2020/138067 (02.07.2020 Gazette 2020/27)**

(54) **THERAPEUTIC AGENT FOR RNA VIRAL INFECTION OBTAINED BY COMBINING A PYRAZINE DERIVATIVE AND A COMPOUND INCREASING THE AMOUNT OF PYRAZINE DERIVATIVE RIBOSE TRIPHOSPHATE IN A CELL.**

THERAPEUTISCHES MITTEL GEGEN RNA-VIRUSINFEKTIONEN, ERHALTEN DURCH DIE KOMBINATION EINES PYRAZINDERIVATS UND EINER VERBINDUNG, DIE DIE MENGE DES PYRAZINDERIVAT-RIBOSETRIPHOSPHATS IN EINER ZELLE ERHÖHT.

AGENT THÉRAPEUTIQUE CONTRE LES INFECTIONS VIRALES À ARN OBTENU PAR COMBINAISON D’UN DÉRIVÉ DE PYRAZINE ET D’UN COMPOSÉ AUGMENTANT LA QUANTITÉ DE DÉRIVÉ DE PYRAZINE- RIBOSE TRIPHOSPHATE DANS UNE CELLULE.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2018 JP 2018240652**

(43) Date of publication of application:
**03.11.2021 Bulletin 2021/44**

(73) Proprietor: **FUJIFILM Toyama Chemical Co., Ltd.**
**Chuo-ku**
**Tokyo 104-0031 (JP)**

(72) Inventors:
- **KOMENO Takashi**
**Toyama-shi, Toyama 930-8508 (JP)**
- **NAKATANI Toshiyuki**
**Ashigarakami-gun, Kanagawa 258-8577 (JP)**
- **KURIMOTO Yusuke**
**Toyama-shi, Toyama 930-8508 (JP)**
- **MATSUDA Shun**
**ashigara-shi, Kanagawa 250-0193 (JP)**
- **BAN Toshikazu**
**Tokyo 107-0052 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 123 276**
**WO-A1-2011/053812**
**JP-A- 2013 509 433**
**US-A1- 2005 119 284**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE BIOSIS [online] BIOSCIENCES INFORMATION SERVICE, PHILADELPHIA, PA, US; February 2004 (2004-02-01), STANGI JASON R ET AL: "Effect of antimetabolite immunosuppressants on flaviviridae, including hepatitis C virus.", XP002805713, Database accession no. PREV200400232583**
- **STANGI JASON R ET AL: "Effect of antimetabolite immunosuppressants on flaviviridae, including hepatitis C virus.", TRANSPLANTATION (HAGERSTOWN), vol. 77, no. 4, February 2004 (2004-02-01), pages 562 - 567, ISSN: 0041-1337**
- **FURUTA Y ET AL: "T-705 (favipiravir) and related compounds: Novel broad-spectrum inhibitors of RNA viral infections", ANTIVIRAL RESEARCH, ELSEVIER BV, NL, vol. 82, no. 3, 1 June 2009 (2009-06-01), pages 95 - 102, XP026018728, ISSN: 0166-3542, [retrieved on 20090306], DOI: 10.1016/J.ANTIVIRAL.2009.02.198**
- **KOTHILA THARMARAJAH ET AL: "Chikungunya: vaccines and therapeutics", F1000RESEARCH, vol. 6, 1 January 2017 (2017-01-01), pages 1 - 7, XP055721824, DOI: 10.12688/f1000research.12461.1**
- **DATABASE BIOSIS [online] BIOSCIENCES INFORMATION SERVICE, PHILADELPHIA, PA, US; February 2004 (2004-02-01), STANGI JASON R ET AL: "Effect of antimetabolite immunosuppressants on flaviviridae, including hepatitis C virus.", XP002805713, Database accession no. PREV200400232583**
- **STANGI JASON R ET AL: "Effect of antimetabolite immunosuppressants on flaviviridae, including hepatitis C virus.", TRANSPLANTATION (HAGERSTOWN), vol. 77, no. 4, February 2004 (2004-02-01), pages 562 - 567, ISSN: 0041-1337**
- **YANG QUAN-EN: "Human immunodeficiency virus reservoir might be actively eradicated as residual malignant cells by cytotoxic chemotherapy", MEDICAL HYPOTHESES, EDEN PRESS, PENRITH, US, vol. 62, no. 3, 1 January 2004 (2004-01-01), pages 358 - 363, XP002350843, ISSN: 0306-9877, DOI: 10.1016/J.MEHY.2003.10.012**
- **FURUTA. YOUSUKE ET AL.: "T-705(favipiravir) and related compounds:Novel broad-spectrum inhibitors of RNA viral infections", ANTIVIRAL RESEARCH, vol. 82, no. 3, 2009, pages 95 - 102, XP026018728, ISSN: 0166-3542**
- **YANG,QUAN-EN ET AL.: "Human immunodeficiency virus reservoir might be actively eradicated as residual malignant cells by cytotoxic chemotherapy", MEDICAL HYPOTHESES, vol. 62, no. 3, 2004, pages 358 - 363, XP002350843, ISSN: 0306-9877, DOI: 10.1016/j.mehy.2003.10.012**
- **ST. GEME JR., J.W. ET AL.: "Disruption of Virus Biosynthesis in vitro with 6-mercaptopurine. I. Effect on DNA- and RNA- Viruses., Proceedings of the Society for Experimental Biology and Medicine", SOCIETY FOR EXPERIMENTAL BIOLOGY AND MEDICINE, vol. 116, 1964, pages 228 - 231, ISSN: 0037-9727**
- **THARMARAJAH KOTHILA, MAHALINGAM SURESH, ZAID ALI: "Chikungunya:vaccines and therapeutics", F1000RESEARCH, vol. 6, 2017, pages 1 - 7, XP055721824, ISSN: 2046-1402**


(52) Cooperative Patent Classification (CPC): (Cont.)

C-Sets
**A61K 31/495, A61K 2300/00;**
**A61K 31/4965, A61K 2300/00;**
**A61K 31/519, A61K 2300/00;**
**A61K 31/52, A61K 2300/00;**
**A61K 31/522, A61K 2300/00;**
**A61K 31/7056, A61K 2300/00**

## Description

### Technical Field

The present invention relates to a therapeutic agent for use in treating an RNA viral infection, which comprises a combination of a pyrazine derivative or a salt thereof and one or more types of compounds selected from the group consisting of an antifolate, a thiopurine antimetabolite, thiazofurin, an alkylating agent, and a xanthine derivative which increase the amount of a pyrazine derivative ribose triphosphate in a cell.

### Background Art

RNA viruses such as influenza virus or Ebola virus cause various infectious diseases, and thus, countermeasures for these RNA viruses have been required. Meanwhile, as compounds having a wide range of antiviral activities against many RNA viruses, pyrazine derivatives such as Favipiravir (hereinafter also referred to as "T-705") have been known (Non-Patent Document 1). It was known that T-705 and T-1105 are effective against RNA viral infections (Non-Patent Document 3), and that these two compounds inhibit the replication of Chikungunya virus (a RNA virus) in vitro and that they have beneficial effects on mice infected with this virus (less severe neurological effects and reduction in mortality rate) (Non-Patent Document 4).

If the activities of these pyrazine derivatives were reinforced, the pyrazine derivatives would be useful as medicaments. To date, it has been reported that a combination of Favipiravir and a neuraminidase inhibitor exhibits synergistic effects against influenza virus (Patent Document 1). In addition, it has also been reported that a combination of Favipiravir and Gemcitabine or Obatoclax exhibits synergistic effects against Ebola virus (Patent Document 2). Further, it was known that azathioprine, 6-thioguanine as well as methotrexate have either a direct anti-viral effect or have a beneficial effect when treating viral infections (Non-Patent Documents 5 and 6; Patent Document 3).

However, such RNA viruses acquire drug resistance as a result of evolution. For example, an influenza virus that is resistant to the neuraminidase inhibitor has been known (Non-Patent Document 2). In order to cope with such acquisition of resistance, a novel combination of compounds, which exhibits effects against RNA viruses, has always been required.

As mentioned above, Favipiravir is characterized in that it exhibits a wide range of effects against many RNA viruses. However, a combination of a pyrazine derivative and a specific compound, which is capable of simultaneously reinforcing antiviral activities against a plurality of RNA viruses, has not yet been known.

### Prior Art Documents

#### Patent Documents

Patent Document 1: International Publication WO2008/099874
Patent Document 2: International Publication WO2017/202789
Patent Document 3: International Publication WO 2011/053812

#### Non-Patent Documents

Non-Patent Document 1: Proc Jpn Acad Ser B Phys Biol Sci. 93, 449-463.
Non-Patent Document 2: CDC Health Advisory:CDC issues interim recommendations for the use of influenza antivirals in the setting of oseltamivir Resistance among circulating influenza A (H1N1) viruses, 2008-09 Season.
Non-Patent Document 3: Y. Furuta et.al., Antiviral Research, 82, 95-102 (2009).
Non-Patent Document 4: K. Tharmarajah et al. F1000Research, 6, 2114 (2017).
Non-Patent Document 5: J. R. Stangl et al., CLINICAL TRANSPLANTATION, 77, 562-567 (2004).
Non-Patent Document 6: Q. Yang, Medical Hypothesis, 62, 358-363 (2004).

### Summary of Invention

#### Object to be Solved by the Invention

It is an object of the present invention to provide a therapeutic agent for use in treating an RNA viral infection,

comprising a novel combination of a pyrazine derivative and one or more types of a specific compounds selected from the group consisting of an antifolate, a thiopurine antimetabolite, thiazofurin, an alkylating agent, and a xanthine derivative, which exhibit effects against an RNA virus. In addition, it is another object of the present invention to provide a therapeutic agent for use in treating an RNA viral infection, comprising a combination of a pyrazine derivative and one or more types of a specific compounds selected from the group consisting of an antifolate, a thiopurine antimetabolite, thiazofurin, an alkylating agent, and a xanthine derivative, which are capable of simultaneously reinforcing antiviral activities against a plurality of RNA viruses.

Means for Solving the Object

Under such circumstances, the present inventor has conducted intensive studies. As a result, the present inventor has found that antiviral activities against a plurality of RNA viruses are simultaneously reinforced, when a pyrazine derivative represented by the following formula [1] or a salt thereof:

[Formula 1]

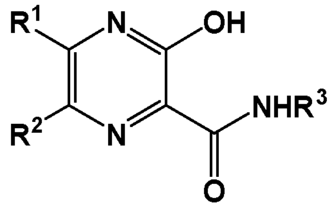

[1]

wherein $R^1$ and $R^2$, which are the same or different, each represent a hydrogen atom or a halogen atom; and $R^3$ represents a hydrogen atom or an amino-protecting group,
is used in combination with a compound which increases the amount of a pyrazine derivative ribose triphosphate in a cell, and in particular, in combination with one or more types of compounds selected from the group consisting of an antifolate, a thiopurine antimetabolite, thiazofurin, an alkylating agent, and a xanthine derivative, thereby completing the present invention.

Specifically, the present invention is as set out in the appended claims and provides the following features:

[1] A therapeutic agent for use in treating an RNA viral infection, which comprises a combination of a pyrazine derivative represented by the following formula [1] or a salt thereof and one or more types of compounds selected from the group consisting of an antifolate, a thiopurine antimetabolite, thiazofurin, an alkylating agent, and a xanthine derivative:

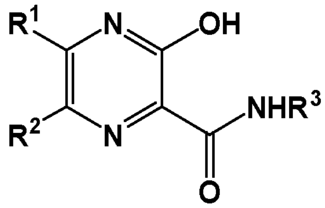

[1]

wherein $R^1$ and $R^2$, which are the same or different, each represent a hydrogen atom or a halogen atom; and $R^3$ represents a hydrogen atom or an amino-protecting group, and
wherein

the antifolate is methotrexate or pralatrexate, the thiopurine antimetabolite is a mercaptopurine derivative represented by the following formula [2]:

[Formula 2]

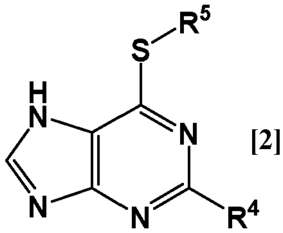
[2]

wherein $R^4$ represents a hydrogen atom or an optionally protected amino group; and $R^5$ represents a hydrogen atom or the following formula [3]:

[Formula 3]

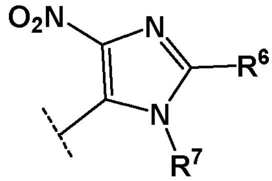
[3]

(wherein $R^6$ represents a hydrogen atom, a $C_{1-6}$ alkyl group, or a carboxy group; and $R^7$ represents a hydrogen atom, a $C_{1-6}$ alkyl group, a benzyl group, or a p-nitrobenzyl group), the alkylating agent is temozolomide, and the xanthine derivative is theophylline.

[2] A therapeutic agent for use in treating an RNA viral infection, comprising a pyrazine derivative represented by the following formula [1] or a salt thereof, which is used in combination with one or more types of compounds selected from the group consisting of an antifolate, a thiopurine antimetabolite, thiazofurin, an alkylating agent, and a xanthine derivative:

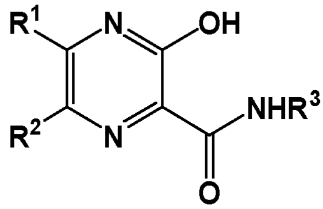
[1]

wherein $R^1$ and $R^2$, which are the same or different, each represent a hydrogen atom or a halogen atom; and $R^3$ represents a hydrogen atom or an amino-protecting group, and
wherein

the antifolate is methotrexate or pralatrexate, the thiopurine antimetabolite is a mercaptopurine derivative represented by the following formula [2]:

[Formula 2]

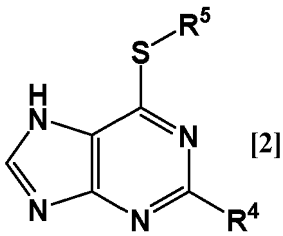
[2]

wherein $R^4$ represents a hydrogen atom or an optionally protected amino group; and $R^5$ represents a hydrogen atom or the following formula [3]:

[Formula 3]

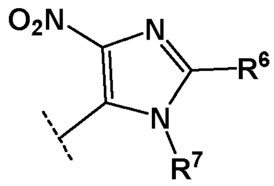

[3]

(wherein $R^6$ represents a hydrogen atom, a $C_{1-6}$ alkyl group, or a carboxy group; and $R^7$ represents a hydrogen atom, a $C_{1-6}$ alkyl group, a benzyl group, or a p-nitrobenzyl group), the alkylating agent is temozolomide, and the xanthine derivative is theophylline.

Advantageous Effects of Invention

A therapeutic agent for use in treating an RNA viral infection, in which a pyrazine derivative or a salt thereof is combined with one or more types of compounds selected from the group consisting of an antifolate, a thiopurine antimetabolite, thiazofurin, an alkylating agent, and a xanthine derivative which increase the amount of a pyrazine derivative ribose triphosphate in a cell, is useful for the treatment, such as therapy or prevention, of RNA viral infection.

Brief Description of Drawings

Fig. 1 is a view showing a change in the amount of T-705-RTP in 293T cells caused by the combined use of T-705 and 6-mercaptopurine (6MP), which is shown in Test Example 6.

Embodiment of Carrying out the Invention

Hereinafter, the present invention will be described in detail.

The term "halogen atom" is used to mean a fluorine atom, a chlorine atom, a bromine atom, and an iodine atom. The term "$C_{1-6}$ alkyl group" is used to mean linear or branched $C_{1-6}$ alkyl groups, such as methyl, ethyl, propyl, isopropyl, butyl, sec-butyl, isobutyl, tert-butyl, pentyl, isopentyl, 2-methylbutyl, 2-pentyl, 3-pentyl and hexyl groups.

Examples of the amino-protecting group may include all groups that can be used as common amino-protecting groups, and examples of the amino-protecting group may include the groups described in, for example, W. Greene et al., Protective Groups in Organic Synthesis, 5th edition, pp. 895 to 1193, 2014, John Wiley & Sons, INC.

Specific examples may include an acyl group, an alkyloxycarbonyl group, an arylalkyloxycarbonyl group, an aryloxycarbonyl group, an arylalkyl group, an alkoxyalkyl group, an arylalkyloxyalkyl group, an arylthio group, an alkylsulfonyl group, an arylsulfonyl group, a dialkylaminoalkylidene group, an arylalkylidene group, a nitrogen-containing heterocyclic alkylidene group, a cycloalkylidene group, a diarylphosphoryl group, a diarylalkylphosphoryl group, an oxygen-containing heterocyclic alkyl group, and a substituted silyl group.

The salt of the compound represented by the formula [1] may be generally known salts of hydroxyl groups. Examples of such salts may include: salts with alkali metals such as sodium and potassium; salts with alkaline earth metals such as calcium and magnesium; ammonium salts; and salts with nitrogen-containing organic bases such as trimethylamine, triethylamine, tributylamine, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine. The salts are preferably pharmacologically acceptable salts, and are more preferably salts with sodium.

In the compound represented by the formula [1], preferably, $R^1$ is a hydrogen atom; $R^2$ is a fluorine atom; and $R^3$ is a hydrogen atom. Besides, this compound is preferably T-705.

Otherwise, in the compound represented by the formula [1], preferably, $R^1$ is a hydrogen atom; $R^2$ is a hydrogen atom; and $R^3$ is a hydrogen atom. This compound is preferably T-1105.

The compound represented by the formula [1] is produced by combining known methods with one another. The compound represented by the formula [1] can be produced by the production method described, for example, in International Publication WO00/10569.

It has been known that the pyrazine derivative represented by the formula [1] or a salt thereof, for example, T-705, undergoes ribosyl phosphorylation in a cell, and that a pyrazine derivative ribose triphosphate generated as a result of the ribosyl phosphorylation exhibits antiviral action (Antimicrob Agents Chemother. 2005; 49(3): 981-6.). Herein, the pyrazine derivative ribose triphosphate is a compound represented by the following formula [4]:

[Formula 4]

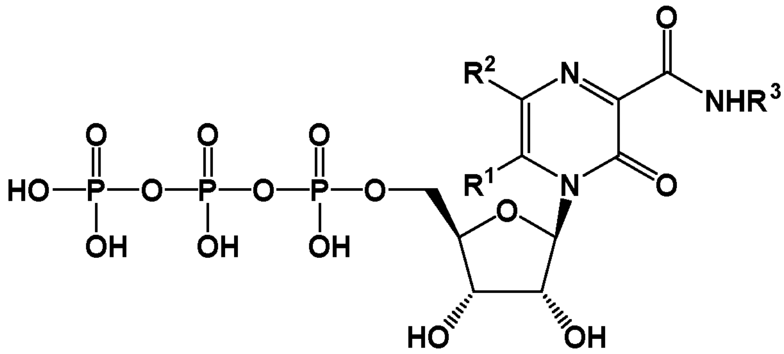

[4]

wherein $R^1$ and $R^2$, which are the same or different, each represent a hydrogen atom or a halogen atom; and $R^3$ represents a hydrogen atom or an amino-protecting group.

The term "compound which increases the amount of a pyrazine derivative ribose triphosphate in a cell" is a compound that activates or inhibits enzymes or metabolic pathways in a cell when the compound is used in combination with the pyrazine derivative represented by the formula [1] or a salt thereof, so that the compound increases the amount of a pyrazine derivative ribose triphosphate in the cell. When compared with a case where the compound is not combined with the pyrazine derivative represented by the formula [1] or a salt thereof, the compound increases the concentration of the pyrazine derivative ribose triphosphate by preferably 1.5 times or more, and more preferably by 2 times or more, when it is combined with the pyrazine derivative represented by the formula [1] or a salt thereof. Such a compound is an antifolate, a thiopurine antimetabolite, thiazofurin, an alkylating agent, and a xanthine derivative. It is to be noted that the antifolate, the thiopurine antimetabolite and the tiazofurin are all antimetabolites.

The antifolate is a compound that inhibits folate metabolizing enzymes necessary for the synthesis of DNA, and thus suppresses cell proliferation. The antifolate is methotrexate and pralatrexate.

The antifolate may be produced by combining known methods with one another, or a commercially available antifolate may be used.

The thiopurine antimetabolite is an antimetabolite having a thiopurine skeleton, such as 6-mercaptopurine, azathioprine, and 6-thioguanine. The thiopurine antimetabolite is represented by the formula [2]. In the compound represented by the formula [2], preferably, $R^4$ is a hydrogen atom or an amino group, and $R^5$ is a hydrogen atom or a group represented by the formula [3]. In the group represented by the formula [3], preferably, $R^6$ is a hydrogen atom, and $R^7$ is a methyl group.

Examples of the particularly preferred thiopurine antimetabolite may include 6-mercaptopurine, azathioprine, and 6-thioguanine. Among these, 6-mercaptopurine is most preferable.

The thiopurine antimetabolite may be produced by combining known methods with one another, or a commercially available thiopurine antimetabolite may be used.

The alkylating agent is a compound that interacts with DNA and inhibits cell proliferation. The alkylating agent is temozolomide .

The alkylating agent may be produced by combining known methods with one another, or a commercially available alkylating agent may be used.

The xanthine derivative is a compound having a xanthine skeleton. The xanthine derivative is theophylline.

The xanthine derivative may be produced by combining known methods with one another, or a commercially available xanthine derivative may be used.

In the present invention, a pyrazine derivative is used in combination with one or more types of compounds selected from the group consisting of an antifolate, a thiopurine antimetabolite, thiazofurin, an alkylating agent, and a xanthine derivative which increase the amount of a pyrazine derivative ribose triphosphate in a cell. The combination includes a form in which a pyrazine derivative and said compounds which increase the amount of a pyrazine derivative ribose triphosphate in a cell are administered, simultaneously, separately, or in a specific order (a combined use), and a form as a mixture (a compounding agent).

That is to say, the "combined use" does not only mean that the administration period of the pyrazine derivative or a salt thereof is identical to the administration period of the compounds which increase the amount of a pyrazine derivative ribose triphosphate in a cell, but the "combined use" also includes a form in which the pyrazine derivative or a salt thereof and the compounds which increase the amount of a pyrazine derivative ribose triphosphate in a cell are administered in one administration schedule. The administration route of the pyrazine derivative or a salt thereof may be either identical to

or different from the administration route of the compounds which increase the amount of a pyrazine derivative ribose triphosphate in a cell.

The amount ratio between the pyrazine derivative or a salt thereof and the compounds which increase the amount of a pyrazine derivative ribose triphosphate in a cell may be an amount ratio, in which the antiviral activity of the pyrazine derivative or a salt thereof is reinforced. The pyrazine derivative or a salt thereof : the compounds which increase the amount of a pyrazine derivative ribose triphosphate in a cell (molar ratio) is preferably 1 : 500 to 500 : 1, more preferably 1 : 200 to 200 : 1, further preferably 1 : 50 to 50 : 1, and still further preferably 1 : 10 to 10 : 1.

When the inventive pyrazine derivative or a salt thereof and the inventive compounds which increase the amount of a pyrazine derivative ribose triphosphate in a cell are used, in general, pharmaceutical aids used in formulation, such as excipients, carriers, and diluents, may be mixed, as appropriate, into the pyrazine derivative or a salt thereof and the compounds which increase the amount of a pyrazine derivative ribose triphosphate in a cell. The thus obtained mixtures may be processed into tablets, capsules, powder agents, syrups, granules, pills, suspending agents, emulsions, liquid agents, powdery preparations, suppositories, eye drops, nasal drops, ear drops, patches, ointments, injections, etc. according to ordinary methods.

**In** the case of using as a compounding agent, the pyrazine derivative or a salt thereof may be mixed with the compounds which increase the amount of a pyrazine derivative ribose triphosphate in a cell in the above-described formulation process, and they may be homogenized, and may be then processed into a suitable preparation.

The administration route of the therapeutic agent for use in treating an RNA viral infection of the present invention is not particularly limited, and the present therapeutic agent for use in treating an RNA viral infection may be administered via intravenous, oral, intramuscular, subcutaneous, inhalation, spraying or other administration routes. Moreover, the pyrazine derivative or a salt thereof and the compounds which increase the amount of a pyrazine derivative ribose triphosphate in a cell may be administered, simultaneously or in a specific order.

The administration method, the applied dose, and the number of administrations can be selected, as appropriate, depending on the age, body weight, and symptoms of a patient. In general, the pyrazine derivative or a salt thereof used as an active ingredient may be administered to an adult at a dose of 0.1 to 1000 mg/kg, preferably 0.1 to 100 mg/kg, via oral administration or parenteral administration (for example, injection, drip infusion, administration to a rectal site, etc.), once a day or divided over several administrations.

The therapeutic agent for use in treating an RNA viral infection of the present invention is useful for the treatment, such as therapy or prevention, of RNA viral infection.

The RNA viral infection is an infectious disease caused by RNA viruses such as influenza virus, parainfluenza virus, bunyavirus (Crimean-Congo fever virus, Rift Valley fever virus, Lacrosse encephalitis virus, Dobrava virus, Maporal virus, Prospect Hill virus, Andes virus, Sand fly fever virus, Heartland virus, Puntatrovirus, Severe febrile thrombocytopenia virus, etc.), Arenavirus (Funin virus, Pitinde virus, Tacaribe virus, Guanarito virus, Machupo virus, Lymphocytic choriomeningitis virus, Lassa fever virus, etc.), filovirus (Ebola virus, Marburg virus, etc.), rabies virus, human metapneumovirus, RS virus, Nipah virus, Hendra virus, measles virus, hepatitis A virus, hepatitis C virus, hepatitis E virus, Chikungunya virus, Western equine encephalitis virus, Venezuelan encephalitis virus, Eastern equine encephalitis virus, Norovirus, Poliovirus, Echovirus, Coxsackie virus, Enterovirus, Rhinovirus, Rotavirus, Newcastle disease virus, Mumps virus, vesicular stomatitis virus, Japanese encephalitis virus, tick-borne flavivirus, yellow fever virus, dengue virus, West Nile virus, or Zika virus. The present invention can be used preferably as a therapeutic agent for infectious diseases caused by influenza virus, rabies virus, Lassa fever virus, bunyavirus (Crimea-Congo fever virus, Rift Valley fever virus, Severe febrile thrombocytopenia virus, etc.) and filovirus (Ebola virus, Marburg virus, etc.), and particularly preferably as a therapeutic agent for influenza infection.

The therapeutic agent for use in treating an RNA viral infection of the present invention can be further used in combination with other therapeutic agents for RNA viral infection or drugs which exhibit RNA virus inhibitory action, for the purpose of reinforcing the action thereof. Such other therapeutic agents for RNA viral infection are therapeutic agents for RNA viral infection, and examples thereof may include a therapeutic agent for influenza, a therapeutic agent for hepatitis C, and a therapeutic agent for filovirus infection. Examples of the therapeutic agent for influenza may include Amantadine, Rimantadine, Oseltamivir, Zanamivir, Peramivir, Laninamivir, and Baloxavir. Examples of the therapeutic agent for hepatitis C may include Ribavirin, pegylated interferon, Telaprevir, and Boceprevir. Examples of the therapeutic agent for filovirus infection may include Ribavirin, Palivizumab, Motabizumab, RSV-IGIV, MEDI-557, A-60444, MDT-637, BMS-433771, Amiodarone, Dronedarone, Verapamil, Ebola convalescent plasma, TKM-100201, BCX4430, FGI-106, TKM-Ebola, ZMapp, rNAPc2, OS-2966, MVA-BN filo, Brincidofovir, and Ad26-ZEBOV. Examples of such drugs which exhibit RNA virus inhibitory action may include mycophenolic acid, Daptomycin, Nicorosamide, Azithromycin, Novobiocin, Chloroquine, Memantine, Prochlorperazine, Chlorcyclizine, Manidipine, GS-5734, Imatinib, Chlorpromazine, and Nitazoxanide (Journal of Young Pharmacists. 2019; 11(2): 117-121.). It is preferable to combine the therapeutic agent for use in treating an RNA viral infection of the present invention with Ribavirin.

When the therapeutic agent for use in treating an RNA viral infection of the present invention is used in combination with other therapeutic agents for RNA viral infection or drugs which exhibit RNA virus inhibitory action,

the amount ratio between the pyrazine derivative or a salt thereof and the one or more types of compounds which increase the amount of a pyrazine derivative ribose triphosphate in a cell is not particularly limited, as long as it is an amount ratio, in which the antiviral activity of the pyrazine derivative or a salt thereof is reinforced. The pyrazine derivative or a salt thereof : the one or more types of compounds which increase the amount of a pyrazine derivative ribose triphosphate in a cell (molar ratio) is preferably 1 : 500 to 500 : 1, more preferably 1 : 250 to 250 : 1, and further preferably 1 : 150 to 150 : 1.

The amount ratio between the therapeutic agent for use in treating an RNA viral infection of the present invention and other therapeutic agents for RNA viral infection or drugs which exhibit RNA virus inhibitory action is not particularly limited, as long as it is an amount ratio, in which the antiviral activity of the therapeutic agent for use in treating an RNA viral infection of the present invention is reinforced. The therapeutic agent for use in treating an RNA viral infection of the present invention : other therapeutic agents for RNA viral infection or drugs which exhibit RNA virus inhibitory action (molar ratio) is preferably 1 : 100 to 100 : 1, more preferably 1 : 50 to 50 : 1, further preferably 1 : 10 to 10 : 1, and still further preferably 1 : 5 to 5 : 1.

## Examples

Next, the present invention will be described in the following examples. However, these examples are not intended to limit the scope of the present invention.

### Test Example 1

The effects of the combination of a pyrazine derivative and an antimetabolite against an RNA virus were examined using the replicon activity of a complex of the virus with RNA-dependent RNA polymerase (RdRp). For evaluation of the replicon activity, a reporter assay system was constructed using cells, with reference to a known method (Neumann, G. et al., J. Virol. 74: 547-551 (2000)). In this assay system, the replicon activity was evaluated using the activity of luciferase (Luc) serving as a reporter protein.

6-Fluoro-3-hydroxy-2-pyrazinecarboxamide (T-705) was selected as a pyrazine derivative. Regarding antimetabolites, methotrexate and pralatrexate were selected as antifolates, and 6-mercaptopurine, azathioprine, and thioguanosine were selected as thiopurine antimetabolites. As an RNA virus, an influenza virus was selected.

#### (1) Construction of reporter assay system

#### (1-1) Cloning of wild-type viral protein genes

In accordance with the method described in the above-described known publication, genes encoding the viral proteins PA, PB1, PB2, and NP were amplified from the Influenza A/PR/8/34 (H1N1) strain by an RT-PCR method, and were then cloned into a pcDNA3.1 vector. The obtained pcDNA3.1/PR8_PA plasmid DNA, pcDNA3.1/PR8_PB1 plasmid DNA, pcDNA3.1/PR8_PB2 plasmid DNA, and pcDNA3.1/PR8_NP plasmid DNA were denominated as wild-type PA DNA, wild-type PB1 DNA, wild-type PB2 DNA, and wild-type NP DNA, respectively.

#### (1-2) Production of reporter plasmid

Subsequently, a plasmid DNA comprising a reporter gene was produced. Specifically, a DNA was produced by connecting, from the 5'-terminus to the 3'-terminus, a region encoding a human-derived polymerase I promoter (Jones, M. H. et al., Proc. Natl. Acad. Sci. USA 85: 669-673 (1988)), a Luc gene sandwiched with the NTRs of the Influenza A/PR/8/34 (H1N1) strain, and a region encoding a mouse-derived polymerase I terminator (Grummt, I. et al., Cell 45: 837-846 (1986)), and the produced DNA was then cloned into a pcDNA3.1 vector to obtain the reporter plasmid pcDNA3.1/polI_NTR_Rh-Luc.

#### (1-3) Adjustment of 293T cells

#### (1-3-1) Culture of 293T cells

As cells used herein, human embryonic kidney cells 293T expressing SV40 large T antigen (hereinafter referred to as "293T cells") were used. 293T cells, which had been sub-cultured in a DMEM medium supplemented with 10% fetal bovine serum under conditions of 5% $CO_2$ at 37°C, were removed by a trypsin ethylenediaminetetraacetate method, and were then seeded in a flask to result in an amount of $2.4 \times 10^7$ cells/225 $cm^2$ flask. Thereafter, the cells were cultured overnight under conditions of 5% $CO_2$ at 37°C, so as to obtain a single layer of 293T cells.

(1-3-2) Transduction into 293T cells

To 3 mL of Opti-MEM (manufactured by Thermo Fisher Scientific), 0.96 μg of wild-type PA DNA, 9.6 μg of wild-type PB1 DNA, 9.6 μg of wild-type PB2 DNA, 9.6 μg of wild-type NP DNA, and 9.6 μg of two types of reporter plasmids pcDNA3.1/poll_NTR_RhLuc, and 9.6 μg of pGL4.54 (manufactured by Promega, #E5061) (for confirmation of the presence or absence of DNA introduction) were added, and further, 49 μL of PLUS Reagent included with Lipofectamine LTX Reagent with PLUS Reagent (manufactured by Thermo Fisher Scientific) was added. At the same time, 0.23 mL of Lipofectamine LTX Reagent was added to 2.9 mL of Opti-MEM, and was then left for 5 minutes. Thereafter, the two liquids were mixed with each other to a volume ratio of 1 : 1, and the obtained mixture was then left at room temperature for 15 minutes. The above mixed solution was added to a culture flask containing 293T cells to result in an amount of 6 mL/225 $cm^2$ flask, and was then left for approximately 6 hours, so that the RdRp of the influenza virus, NP, and the reporter plasmid were introduced into the cells.

(2) Measurement of replicon inhibiting acitvity by combination of T-705 and antimetabolite

(2-1) Addition of drugs to cells and culture

The transduced 293T cells were removed by a trypsin ethylenediaminetetraacetate method, and were then seeded on a 384-well plate (manufactured by Corning, #3707) to which any one of the following test solutions (A) to (D) had been added, so as to achieve $1.5 \times 10^4$ cells/10 μL/well. At the same time, as blanks to which no cells were added, the following solutions (A) to (D) were added to wells, so as to prepare blank wells. Thereafter, the obtained mixtures were each cultured under conditions of 5% $CO_2$ at 37°C for approximately 20 hours.

(A) 10 μM T-705 and a 0.1% DMSO-containing medium
(B) A single antimetabolite agent (final concentration: 10 μM to 0.08 μM; 5-fold serial dilution at a common ratio of 5 from10 μM) and a 0.1% DMSO-containing medium
(C) T-705 (final concentration: 10 μM), an antimetabolite (final concentration: 10 μM to 0.08 μM; 5-fold serial dilution at a common ratio of 5 from10 μM), and a 0.1% DMSO-containing medium
(D) A 0.1% DMSO-containing medium

All of the solutions (A) to (D) were diluted and mixed with a DMEM medium supplemented with 10% fetal bovine serum.

(2-2) Measurement of Luc activity and evaluation of replicon inhibiting activity

For the measurement of Luc activity, Dual-Glo Luciferase Assay System (manufactured by Promega) was used. After completion of the culture for approximately 20 hours, Dual-Glo Reagent included with the aforementioned System was added in an amount of 20 μL/well to the plate, and the obtained mixture was then stirred at room temperature for 10 minutes or more, so that the cells were dissolved. Thereafter, using a microplate reader (manufactured by PerkinElmer, 2104 EnVision), fluorescence intensity was measured. Subsequently, Stop & Glo Reagent included with the System was added in an amount of 20 μL/well to the plate, and the obtained mixture was then stirred at room temperature for 10 minutes or more. Thereafter, using the microplate reader (as described above), fluorescence intensity was measured. The operations were carried out with the number of cases of 1. According to the following equation, a replicon reaction percentage and a replicon inhibiting percentage were calculated.

[Replicon reaction percentage (%) with non-addition of T-705] = [(mean fluorescence intensity in antimetabolite-added well) - (mean fluorescence --> intensity in 0.1% DMSO-containing medium-added well)] / [(mean fluorescence intensity in drug-non-added well) - (mean fluorescence intensity in 0.1% DMSO-containing medium-added well)] × 100

[Replicon reaction percentage (%) with addition of T-705] = [(mean fluorescence intensity in antimetabolite and T-705-added well) - (mean fluorescence intensity in 10 μM T-705 and 0.1% DMSO-containing medium-added well)] / [mean fluorescence intensity in T-705-added well) - (mean fluorescence intensity in 10 μM T-705 and 0.1% DMSO-containing medium-added well)] × 100

Replicon inhibiting percentage (%) = 100-[replicon reaction percentage (%)]

As a result, as shown in Table 1, it was found that the replicon inhibiting percentage of T-705 is improved by addition of an antifolate or a thiopurine preparation, although a single T-705 agent has an uninhibitable concentration. It is to be noted that thioguanosine is a compound formed by binding a ribose ring to 6-thioguanine. Since it is considered that 6-thioguanine is metabolized to thioguanosine (or a metabolite of thioguanosine) in cells, 6-thioguanine is also considered to improve the replicon inhibiting percentage of T-705, as with thioguanosine.

[Table 1]

| Replicon inhibiting percentage (%) of T-705 by combination of T-705 and antimetabolite | | | | | | |
|---|---|---|---|---|---|---|
| Combined drug | T-705 concentration (μM) | Antimetabolite concentration (μM) | | | | |
| | | 0 | 0.08 | 0.4 | 2.0 | 10.0 |
| Methotrexate | 0 | -3.3 | 1.3 | 18 | 20 | 19 |
| | 10 | -7.8 | 11 | 36 | 34 | 37 |
| Pralatrexate | 0 | -2.3 | 24 | 23 | 2 | 23 |
| | 10 | 0.58 | 33 | 33 | 33 | 35 |
| 6-Mercaptopurine | 0 | -2.3 | -1.1 | 1.5 | 2.7 | 8.2 |
| | 10 | 2.2 | 1.8 | 3.7 | 17 | 38 |
| Azathioprine | 0 | -0.98 | 3.1 | -6.1 | 3.4 | 5.8 |
| | 10 | -1.8 | 3.3 | 1.3 | 5.9 | 26 |
| Thioguanine | 0 | -7.5 | -4.8 | -5.4 | -3.7 | 12 |
| | 10 | -2.3 | -1.2 | * | 14 | 23 |
| * Data could not be obtained due to errors. | | | | | | |

(3) Measurement of replicon 50% inhibitory concentration by combination of T-705 and antimetabolite

Using the following test solutions (E) to (H), addition of the drugs to the cells and the culture of the cells were carried out in the same manner as that in (2-1) above. Thereafter, the replicon inhibiting activity was evaluated in the same manner as that in (2-2) above.

(E) A single T-705 agent (final concentration: 1.4 μM to 1000 μM; 8-fold serial dilution at a common ratio of 3 from 1000 μM) and a 0.1% DMSO-containing medium
(F) A single antimetabolite agent (final concentration: 0.03 μM to 200 μM; 10-fold serial dilution at a common ratio of 3 from 200 μM, or 10-fold serial dilution at a common ratio of 2 from 20 μM) and a 0.1% DMSO-containing medium
(G) T-705 (final concentration: 1.4 μM to 1000 μM; 8-fold serial dilution at a common ratio of 3 from 1000 μM), antimetabolite (final concentration: 0.03 μM to 200 μM; 10-fold serial dilution at a common ratio of 3 from 200 μM, or 10-fold serial dilution at a common ratio of 2 from 20 μM), and a 0.1% DMSO-containing medium
(H) A 0.1% DMSO-containing medium

For calculation of the 50% inhibitory concentration, Dose Response One Site (Sigmoidal Dose-Response Model; [fit = (A + ((B-A) / (1 + ((C/x)^D))))]) of XLfit (version 5.3.1.3) was used. The 50% inhibitory concentration of T-705 used in combination with each antimetabolite is shown in Table 2. It was found that the combination of T-705 and each antimetabolite shows a lower 50% inhibitory concentration than a single T-705 agent.

[Table 2]

| | | | | | | | | | | |
|---|---|---|---|---|---|---|---|---|---|---|
| 6-Mercaptopurine concentration [μM] | 0 | 0.03 | 0.09 | 0.27 | 0.82 | 2. 5 | 7. 4 | 22 | 67 | 200 |
| T-705 50% inhibitory concentration [μM] | 310 | 303 | 275 | 219 | 163 | 67 | 16 | 2.3 | 5.1 | 23 |
| Methotrexate concentration [μM] | 0 | 0.08 | 0.16 | 0.31 | 0. 63 | 1.25 | 2.5 | 5 | 10 | 20 |
| T-705 50% inhibitory concentration [μM] | 314 | 46 | 43 | 41 | 47 | 40 | 38 | 39 | 28 | 30 |
| Pralatrexate concentration [μM] | 0 | 0.08 | 0.16 | 0.31 | 0.63 | 1.25 | 2.5 | 5 | 10 | 20 |

(continued)

| | | | | | | | | | | |
|---|---|---|---|---|---|---|---|---|---|---|
| T-705 50% inhibitory concentration [μM] | 304 | 32 | 31 | 25 | 29 | 18 | 24 | 25 | 22 | 23 |

In the present test system, a single 6-mercaptopurine agent (<200 μM), a single methotrexate agent (<20 μM), and a single pralatrexate agent (<20 μM) have a replicon inhibiting percentage of less than 50%, and no inhibitory activity was found. The replicon inhibiting activity was significantly reinforced by the combination of T-705 and an antimetabolite, compared with the single use of T-705.

Test Example 2

Regarding the effects of the combined use of a pyrazine derivative and an antimetabolite, a test was further performed using virus-infected cell models.

T705 was selected as a pyrazine derivative. 6-Mercaptopurine was selected as an antimetabolite. As an RNA virus, an influenza virus was selected.

(1) Culture of MDCK cells

Madin-Darby Caine Kidney cells (hereinafter referred to as "MDCK"), which had been sub-cultured in a 10% fetal bovine serum-added Eagle's MEM medium used as a culture medium under conditions of 5% $CO_2$ at 37°C, were removed according to a trypsin ethylenediaminetetraacetate method. A suspension prepared by allowing 100 μL of the same medium as described above to comprise the $2 \times 10^4$ cells was seeded on a 96-well plate. The suspension was cultured overnight under conditions of 5% $CO_2$ at 37°C, so as to obtain MDCK cells that were in the form of a single layer.

(2) Infection with influenza virus and addition of drug

As a test medium, a medium, which was prepared by adding L-1-tosylamide-2-phenylethylchloromethylketone (TPCK)-treated trypsin to an Eagle's MEM medium supplemented with 60 μg/mL kanamycin to a concentration of 1 μg/mL, was used. A culture supernatant of the MDCK cells obtained in (1) above was removed, and the plate was then rinsed with an Eagle's MEM medium, and the following (A) to (C) were then added to each well. After addition of the drug, the obtained mixture was cultured under conditions of 5% $CO_2$ at 35°C for 3 to 4 days.

(A) 100 μL of an Eagle's MEM medium supplemented with 60 μg/mL kanamycin
(B) 50 μL of an influenza virus (PR/8(H1N1)) solution adjusted to $4.0 \times 10^3$ PFU/mL with an Eagle's MEM medium comprising TPCK-treated trypsin having 4 times the concentration of the test medium
(C) 50 μL of an Eagle's MEM medium comprising combinations of individual set concentrations of T-705 and 6-mercaptopurine each having 4 times the set concentrations T-705 set concentrations (μM): 0, 0.01, 1, 3, 10, and 30 6-Mercaptopurine set concentrations (μM): 0, 0.1, 0.3, 1, 3, and 10

(3) Judgment of cytopathic effect (CPE)

Cytopathic effect (CPE) found with proliferation of the influenza virus was judged by the following method.

After completion of the culture, 50 μL of a 100% formalin solution was added to each well, and thereby, the virus was inactivated and the cells were immobilized. The reaction mixture was left at rest at room temperature for 2 hours or more, and a 0.005% methylene blue solution was then added in an amount of 100 μL/well to each well, followed by leaving at rest at room temperature for 1 hour. Thereafter, the 0.005% methylene blue solution was removed, and the reaction mixture was lightly washed with water and was then air-dried. Thereafter, using a microplate reader (manufactured by Tecan; infinit M200), the absorbance (660 nm) was measured. As a non-infection control, 50 μL of an Eagle's MEM medium comprising TPCK-treated trypsin having 4 times the concentration of the test medium was added to the wells, instead of the influenza virus solution, and the same operations as those for the test group were then carried out, followed by the measurement of the absorbance. As a blank, the same operations as those for the non-infection control were carried out on wells, into which MDCK cells had not been seeded, and the absorbance was then measured.

The test was carried out three times with the number of cases of 1 (the number of cases was 8 in the infection control). Using a mean value, the numerical value obtained by subtracting the blank value from the mean value was defined as an absorbance. The value obtained by subtracting the absorbance of the infection control from the absorbance of the non-infection control was defined as a complete suppression value of virus proliferation. The CPE inhibition percentage in each test was calculated according to the following equation.

Virus proliferation suppressing percentage = [(absorbance upon action of single agent and combined use) - (absorbance of infection control)] / [(absorbance of non-infection control) - (absorbance of infection control)]

For calculation of 50% inhibitory concentration, FORECAST function (linear regression method) of Microsoft Office Excel 2007 was used.

A change in the 50% inhibitory concentration of T-705 upon addition of 6-mercaptopurine is shown in Table 3. When compared with a single T-705 agent, the 50% inhibitory concentration of T-705 that was used in combination with 6-mercaptopurine became a low value. Besides, in the present test system, a single 6-mercaptopurine (10 μM) agent did not exhibit CPE inhibitory effects.

[Table 3]

| Change in 50% inhibitory concentration of T-705 by addition of 6-mercaptopurine | | | | | | |
|---|---|---|---|---|---|---|
| 6-Mercaptopurine concentration [μM] | 0 | 0.1 | 0.3 | 1 | 3 | 10 |
| T-705 50% inhibitory concentration [μM] | 1.97 | 1.83 | 1.32 | 0.77 | 0.70 | 0.38 |

It could be confirmed even by using infected cell models that antiviral activity is reinforced by a combination of T-705 and an antimetabolite, compared with a single T-705 agent.

Test Example 3

Using the same influenza virus-infected cell models as those in Test Example 2, the combined use effects of a pyrazine derivative, an antimetabolite and another therapeutic agent for use in treating an RNA viral infection were further examined.

As a pyrazine derivative, T-705 was selected. As an antimetabolite, 6-mercaptopurine was selected. As another therapeutic agent for use in treating an RNA viral infection, Ribavirin was selected. The set concentrations of the drugs and the judgment of CPE were determined as follows, and the test was then carried out.

The set concentrations of the drugs were determined as follows.

T-705 set concentrations (μM): 0, 0.01, 0.03, 0.1, 0.3, 1, 3, 10, and 30
Ribavirin set concentrations (μM): 0, 0.1, 0.3, 1, 3, 10, 30, and 100
6-Mercaptopurineset concentrations (μM): 0 and 10

With regard to the judgment of CPE, a plate stained with a methylene blue solution was confirmed by visual observation, so that a minimal drug concentration which exhibits a complete CPE suppression effect was determined.

When T-705 and Ribavirin were used each alone, the minimum drug concentrations which exhibit complete CPE suppression effects were 3 and 30 (μM), respectively. In addition, the minimum drug concentration of T-705 which exhibits a complete CPE suppression effect was 0.3 (μM), when 6-mercaptopurine (10 μM) was added. Moreover, the minimum drug concentration of T-705 which exhibits a complete CPE suppression effect was 0.1 (μM), when 6-mercaptopurine (10 μM) and Ribavirin (3 μM) were added.

It could be confirmed using infected cell models that antiviral activity is further reinforced by a combination of T-705, an antimetabolite and another therapeutic agent for use in treating an RNA viral infection, when compared with a single T-705 agent, or a combination of T-705 and an antimetabolite.

Test Example 4

Cell models infected with virus other than the influenza virus were created using Punta Toro virus as a bunyavirus, and the same test as that in Test Example 2 was then carried out using the cell models. T705 was selected as a pyrazine derivative. As compounds, 6-mercaptopurine, azathioprine, temozolomide, theophylline, and tiazofurin were selected.

(1) Culture of 293T cells

As cells used herein, 293T cells were used. 293T cells, which had been sub-cultured in a DMEM medium supplemented with 10% fetal bovine serum under conditions of 5% $CO_2$ at 37°C, were removed by a trypsin ethylenediaminetetraacetate method, and a suspension was then prepared with the same medium as described above, such that 1 mL of the medium comprises $1 \times 10^5$ cells. The prepared suspension was seeded on a 24-well plate, and was then cultured overnight under conditions of 5% $CO_2$ at 37°C.

(2) Infection with Punta Toro virus and addition of drugs

A culture supernatant of the 293T cells obtained in (1) above was removed, and the plate was then rinsed with an Eagle's MEM medium. Thereafter, 0.1 mL of a Punta Toro virus solution adjusted to $1.0 \times 10^3$ PFU/mL was added to each well, and the obtained mixture was then cultured under conditions of 5% $CO_2$ at 37°C for 1 hours. Thereafter, the virus solution was removed, and a 1% fetal bovine serum-added Eagle's MEM medium comprising a single T-705 agent (final concentration: 20 μM), an antimetabolite (final concentration: 10 μM), or a mixture of a single T-705 agent (final concentration: 20 μM) and an antimetabolite (final concentration: 10 μM), was added to each well. The thus obtained mixture was cultured under conditions of 5% $CO_2$ at 37°C for 3 days. In addition, as an infection control, a 1% fetal bovine serum-added Eagle's MEM medium was added, instead of the medium comprising the drugs.

(3) Quantification of Punta Toro virus

(3-1) Culture of Vero cells

Monkey kidney Vero cells, which had been sub-cultured in a 10% fetal bovine serum-added Eagle's MEM medium used as a culture medium under conditions of 5% $CO_2$ at 37°C, were removed according to a trypsin ethylenediaminetetraacetate method. A suspension prepared by allowing 100 μL of a 2% fetal bovine serum-added Eagle's MEM medium to comprise the $2 \times 10^4$ cells was seeded on a 96-well plate. The suspension was cultured overnight under conditions of 5% $CO_2$ at 37°C, so as to obtain Vero cells that were in the form of a single layer.

(3-2) Quantification of virus

The virus culture solution obtained in (2) above was serially diluted up to 5 times with an Eagle's MEM medium at a common ratio of 10. The stock solution and diluted solutions were each added in an amount of 50 μL each to the Vero cells obtained in (3-1) above (n = 4). The obtained mixture was cultured under conditions of 5% $CO_2$ at 37°C for 3 to 4 days.

(3-3) Calculation of virus amount

After completion of the culture, 50 μL of a 100% formalin solution was added to each well, and thereby, the virus was inactivated and the cells were immobilized. The reaction mixture was left at rest at room temperature for 2 hours or more, and a 0.005% methylene blue solution was then added in an amount of 100 μL/well to each well, followed by leaving at rest at room temperature for 1 hour. Thereafter, the 0.005% methylene blue solution was removed, and the resultant was lightly washed with water and was then air-dried. Thereafter, the logarithmic value of the median infective dose of each sample ($LogTCID_{50}$) was calculated using the following equation of a Bohrens-Karber method. The effect of reducing the virus amount with respect to the infection control was indicated with ⊿$LogTCID_{50}$.

$$LogTCID_{50} = D + h \times d + 0.5 \times d$$

D: The common logarithm of the highest dilution factor, at which all of the wells (n = 4) were CPE-positive (positive rate = 1)
h: The CPE-positive rate of each well (in the case of <1)
d: The common logarithm of the dilution factor of the sample

The results are shown in Table 4. It could be confirmed that ⊿ $LogTCID_{50}$ was increased by using T-705 in combination with the compound.

[Table 4]

| | T-705 | 6-Mercaptopurine | Azathioprine | Temozolomide | Theophylline | Tiazofurin |
|---|---|---|---|---|---|---|
| Single use | 1.0* | 0.2 | 1.0 | 0.0 | 1.0 | 1.0 |
| Combined use with T-705 | - | 5.5 | 5.0 | 2.0 | 2.25 | 2.25 |

* The ⊿$LogTCID_{50}$ value that shows the effect of reducing the virus amount with respect to the infection control is shown.

It could be confirmed that antiviral activity is reinforced by the combined use of T-705 and an antimetabolite, not only against the influenza virus of Test Example 2, but also against the Punta Toro virus. In addition, it was found that temozolomide, theophylline and tiazofurin also exhibit an excellent effect of reducing the virus amount, when they are used in combination with T-705.

Test Example 5

T-1105 was selected as a pyrazine derivative, 6-mercaptopurine was selected as a compound, and the same test as that in Test Example 4 was carried out. The results are shown in Table 5.

[Table 5]

| | T-1105 | 6-Mercaptopurine |
|---|---|---|
| Single use | 0.0 | 0.0 |
| Combined use with T-1105 | - | 2.5 |

It could be confirmed that, as with T-705, the antiviral effects of T-1105 are also reinforced by being used in combination with the antimetabolite.

Test Example 6

As described above, the pyrazine derivative or a salt thereof undergoes ribosyl phosphorylation in a cell. For example, it has been known that T-705-4-ribofuranosyl-5-triphosphate (T-705RTP) generated as a result of the ribosyl phosphorylation of T-705 inhibits the RdRp protein of virus and thereby exhibits antiviral activity. In order to examine whether or not the effect of 6-mercaptopurine to reinforce the activity of T-705, which was shown in Test Example 1, is caused by a change in the amount of T-705RTP, the amount of T-705RTP in 293T cells was measured under the treatment with 6-mercaptopurine.

(1) Measurement of T-705RTP in 293T cells under treatment of 293T cells with 6-mercaptopurine

(1-1) Extraction of intracellular T-705RTP

293T cells ($5 \times 10^5$ cells) seeded in each well of a 6-well plate were cultured with a vehicle (0.3% DMSO) or with 6-mercaptopurine, T-705, or the two drugs in various concentrations, under conditions of 5% $CO_2$ at 37°C for 24 hours.

After completion of the culture, the reaction mixture was washed with 2 ml of PBS, and 300 μl of trypsin was then added thereto. The obtained mixture was left at rest at 37°C for 5 minutes, and the cells were then removed. Subsequently, 400 μl of a trypsin inhibitor was added and suspended into the cells, and the cell suspension was then recovered in a 1.5-ml tube. The cell suspension was centrifuged at 1,000 rpm at 4°C for 2 minutes (MX-301, TOMY), and 500 μl of a supernatant was then removed. Subsequently, 1 ml of PBS was added and re-suspended into the cells, and 60 μl of the suspension was then distributed in a 1.5-ml tube and was used as a sample for the measurement of the number of distributed cells. Then, 1 ml of the remaining cell suspension was distributed in a 1.5-ml tube, and was then used for the extraction of T-705RTP. The distributed cell suspension was centrifuged at 2,000 × g at 4°C for 2 minutes, and 900 μl of a supernatant was then removed. Subsequently, for the extraction of T-705RTP, 500 μl of methanol was added to the suspension, and was fully suspended. Using a centrifugal evaporator (CVE-3100, EYELA), the obtained suspension was evaporated and dried at 37°C. The sample was then used in an LC/MS/MS analysis.

(1-2) Measurement of number of cells and LC/MS/MS analysis

The measurement of the number of cells and an LC/MS/MS analysis were carried out, and the amount of T-705RTP in each sample was then measured. The test method was applied in accordance with Matsuda S, Kasahara T. Genes Environ. 40: 13. (2018). A change in the amount of T-705RTP is shown in Fig. 1. It was found that the amount of T-705RTP in the cells after the treatment of T-705 was increased by 6-mercaptopurine.

The mechanism of action of T-705, namely, the mechanism by which after T-705 is converted to T705RTP in a cell, it exhibits antiviral activity, is applied to all species of viruses, which are affected by T-705. Since the amount of T-705RTP in a cell has been increased by an antimetabolite, it is said that the combination of T-705 and an antimetabolite simultaneously reinforces antiviral activities against a plurality of RNA viruses that are affected by T-705.

This mechanism is broadly applied, not only to T-705, but also to the pyrazine derivative represented by the

formula [1] or a salt thereof. For example, it is suggested that a compound represented by the formula [1] other than T-705, wherein $R^1$ is a hydrogen atom, $R^2$ is a hydrogen atom and $R^3$ is a hydrogen atom (i.e., 3-hydroxy-pyrazinecarboxamide), exhibits antiviral activity (Antiviral Res. 2009; 82(3): 95-102.), and that this compound is converted to a ribose triphosphate in a cell (Mol Pharmacol. 2013; 84(4): 615-29.). That is to say, by combining the pyrazine derivative or a salt thereof with one or more types of compounds which increase the amount of a pyrazine derivative ribose triphosphate, antiviral activities against a plurality of RNA viruses that are affected by the pyrazine derivative or a salt thereof can be simultaneously reinforced.

Industrial Applicability

A therapeutic agent for use in treating an RNA viral infection, which comprises a combination of a pyrazine derivative or a salt thereof and one or more types of compounds selected from the group consisting of an antifolate, a thiopurine antimetabolite, thiazofurin, an alkylating agent, and a xanthine derivative, exhibits reinforced antiviral activity, and is useful in the field of pharmaceutical industry.

## Claims

1. A therapeutic agent for use in treating an RNA viral infection, which comprises a combination of a pyrazine derivative represented by the following formula [1] or a salt thereof and one or more types of compounds selected from the group consisting of an antifolate, a thiopurine antimetabolite, thiazofurin, an alkylating agent, and a xanthine derivative:

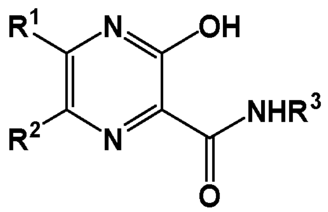
[1]

wherein $R^1$ and $R^2$, which are the same or different, each represent a hydrogen atom or a halogen atom; and $R^3$ represents a hydrogen atom or an amino-protecting group, and
wherein

the antifolate is methotrexate or pralatrexate, the thiopurine antimetabolite is a mercaptopurine derivative represented by the following formula [2]:

[Formula 2]

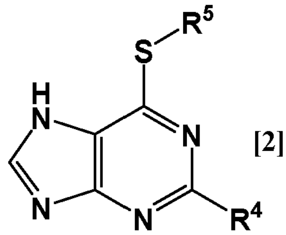
[2]

wherein $R^4$ represents a hydrogen atom or an optionally protected amino group; and $R^5$ represents a hydrogen atom or the following formula [3]:

[Formula 3]

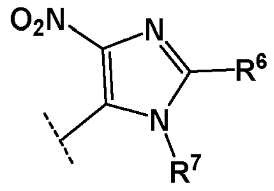

[3]

(wherein $R^6$ represents a hydrogen atom, a $C_{1-6}$ alkyl group, or a carboxy group; and $R^7$ represents a hydrogen atom, a $C_{1-6}$ alkyl group, a benzyl group, or a p-nitrobenzyl group), the alkylating agent is temozolomide, and the xanthine derivative is theophylline.

2. The therapeutic agent for use according to claim 1, wherein $R^1$ represents a hydrogen atom, $R^2$ represents a fluorine atom or a hydrogen atom, and $R^3$ represents a hydrogen atom.

3. The therapeutic agent for use according to claim 2, wherein $R^1$ represents a hydrogen atom, $R^2$ represents a fluorine atom, and $R^3$ represents a hydrogen atom.

4. The therapeutic agent for use according to claim 1, wherein the thiopurine antimetabolite is 6-mercaptopurine, azathioprine, or 6-thioguanine.

5. The therapeutic agent for use according to any one of claims 1 to 4, which further comprises one or more of other therapeutic agents for RNA viral infection or drugs which exhibit RNA virus inhibitory action.

6. The therapeutic agent for use according to claim 5, wherein another therapeutic agents for RNA viral infection or another drug which exhibits RNA virus inhibitory action is Ribavirin.

7. A therapeutic agent for use in treating an RNA viral infection, comprising a pyrazine derivative represented by the following formula [1] or a salt thereof, which is used in combination with one or more types of compounds selected from the group consisting of an antifolate, a thiopurine antimetabolite, thiazofurin, an alkylating agent, and a xanthine derivative:

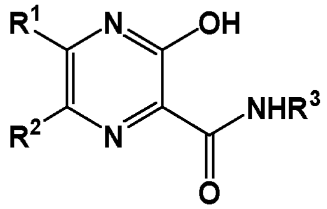

[1]

wherein $R^1$ and $R^2$, which are the same or different, each represent a hydrogen atom or a halogen atom; and $R^3$ represents a hydrogen atom or an amino-protecting group, and
wherein

the antifolate is methotrexate or pralatrexate, the thiopurine antimetabolite is a mercaptopurine derivative represented by the following formula [2]:

[Formula 2]

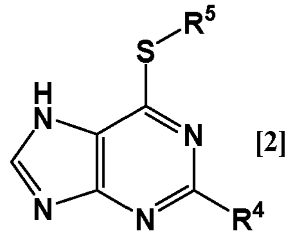

[2]

wherein $R^4$ represents a hydrogen atom or an optionally protected amino group; and $R^5$ represents a hydrogen atom or the following formula [3]:

[Formula 3]

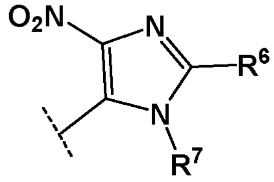

[3]

(wherein $R^6$ represents a hydrogen atom, a $C_{1-6}$ alkyl group, or a carboxy group; and $R^7$ represents a hydrogen atom, a $C_{1-6}$ alkyl group, a benzyl group, or a p-nitrobenzyl group), the alkylating agent is temozolomide, and the xanthine derivative is theophylline.

**8.** The therapeutic agent for use according to claim 7, wherein $R^1$ represents a hydrogen atom, $R^2$ represents a fluorine atom or a hydrogen atom, and $R^3$ represents a hydrogen atom.

**9.** The therapeutic agent for use according to claim 8, wherein $R^1$ represents a hydrogen atom, $R^2$ represents a fluorine atom, and $R^3$ represents a hydrogen atom.

**10.** The therapeutic agent for use according to any one of claims 7 to 9, which is further used in combination with one or more of other therapeutic agents for RNA viral infection or drugs which exhibit RNA virus inhibitory action.

**11.** The therapeutic agent for use according to claim 10, wherein another therapeutic agents for RNA viral infection or another drug which exhibits RNA virus inhibitory action is Ribavirin.

**Patentansprüche**

**1.** Therapeutisches Mittel zur Verwendung in der Behandlung einer RNA-Virusinfektion, welches eine Kombination eines durch die folgende Formel [1] dargestellten Pyrazinderivats oder eines Salzes hiervon und einer oder mehr Arten von Verbindungen umfasst, ausgewählt aus der Gruppe bestehend aus einem Antifolat, einem Thiopurin-Antimetaboliten, Thiazofurin, einem Alkylierungsmittel und einem Xanthinderivat:

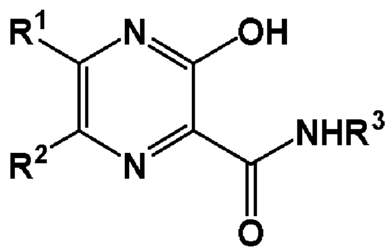

[1]

worin $R^1$ und $R^2$, die gleich oder verschieden sind, jeweils ein Wasserstoffatom oder ein Halogenatom darstellen; und $R^3$ ein Wasserstoffatom oder eine AminoSchutzgruppe darstellt, und
worin
das Antifolat Methotrexat oder Pralatrexat ist, der Thiopurin-Antimetabolit ein Mercaptopurinderivat, dargestellt durch die folgende Formel [2], ist:

[Formel 2]

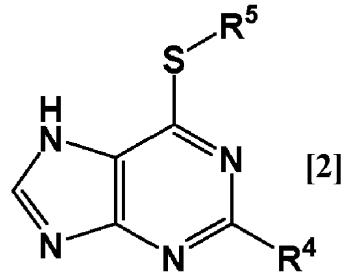

[2]

worin $R^4$ ein Wasserstoffatom oder eine optional geschützte Aminogruppe ist; und $R^5$ ein Wasserstoffatom oder die folgende Formel [3] darstellt:

[Formel 3]

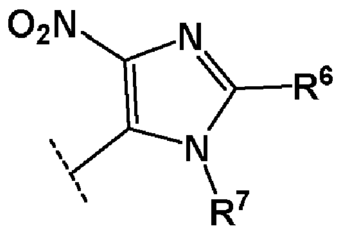

[3]

(worin $R^6$ ein Wasserstoffatom, eine $C_{1-6}$-Alkylgruppe oder eine Carboxygruppe darstellt; und $R^7$ ein Wasserstoffatom, eine $C_{1-6}$-Alkylgruppe, eine Benzylgruppe oder eine p-Nitrobenzylgruppe darstellt), das Alkylierungsmittel Temozolomid ist und das Xanthinderivat Theophyllin **ist.**

2. Therapeutisches Mittel zur Verwendung gemäß Anspruch **1,** worin $R^1$ ein Wasserstoffatom darstellt, $R^2$ ein Fluoratom oder ein Wasserstoffatom darstellt und $R^3$ ein Wasserstoffatom darstellt.

3. Therapeutisches Mittel zur Verwendung gemäß Anspruch **2,** worin $R^1$ ein Wasserstoffatom darstellt, $R^2$ ein Fluoratom darstellt und $R^3$ ein Wasserstoffatom darstellt.

4. Therapeutisches Mittel zur Verwendung gemäß Anspruch 1, worin der Thiopurin-Antimetabolit 6-Mercaptopurin, Azathioprin oder 6-Thioguanin ist.

5. Therapeutisches Mittel zur Verwendung gemäß irgendeinem der Ansprüche 1 bis 4, welches ferner ein oder mehr andere therapeutische Mittel für RNA-Virusinfektionen oder Wirkstoffe, die eine inhibierende Wirkung auf einen RNA-Virus ausüben, umfasst.

6. Therapeutisches Mittel zur Verwendung gemäß Anspruch 5, worin ein anderes therapeutisches Mittel für eine RNA-Virusinfektion oder ein anderer Wirkstoff, der eine inhibierende Wirkung auf einen RNA-Virus ausübt, Ribavirin ist.

7. Therapeutisches Mittel zur Verwendung in der Behandlung einer RNA-Virusinfektion, umfassend ein durch die folgende Formel [1] dargestelltes Pyrazinderivat oder ein Salz hiervon, welches in Kombination mit einer oder mehr Arten von Verbindungen eingesetzt wird, ausgewählt aus der Gruppe bestehend aus einem Antifolat, einem Thiopurin-Antimetaboliten, Thiazofurin, einem Alkylierungsmittel und einem Xanthinderivat:

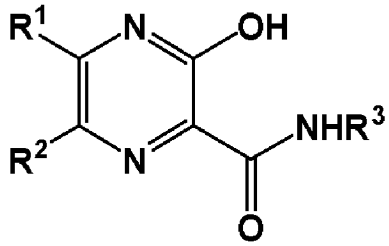

[1]

worin $R^1$ und $R^2$, die gleich oder verschieden sind, jeweils ein Wasserstoffatom oder ein Halogenatom darstellen; und $R^3$ ein Wasserstoffatom oder eine AminoSchutzgruppe darstellt, und worin das Antifolat Methotrexat oder Pralatrexat ist, der Thiopurin-Antimetabolit ein Mercaptopurinderivat, dargestellt durch die folgende Formel [2]:

[Formel 2]

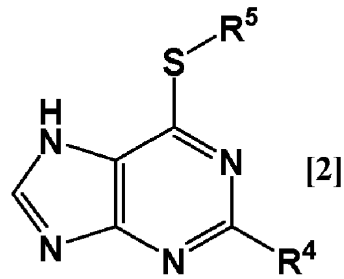

[2]

ist, worin $R^4$ ein Wasserstoffatom oder eine optional geschützte Aminogruppe darstellt; und $R^5$ ein Wasserstoffatom oder die folgende Formel [3] darstellt:

[Formel 3]

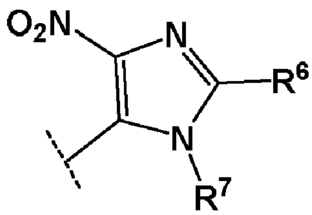

[3]

(worin $R^6$ ein Wasserstoffatom, eine $C_{1-6}$-Alkylgruppe oder eine Carboxygruppe darstellt; und $R^7$ ein Wasserstoffatom, eine $C_{1-6}$-Alkylgruppe, eine Benzylgruppe oder eine p-Nitrobenzylgruppe darstellt), das Alkylierungsmittel Temozolomid ist und das Xanthinderivat Theophyllin ist.

**8.** Therapeutisches Mittel zur Verwendung gemäß Anspruch 7, worin $R^1$ ein Wasserstoffatom darstellt, $R^2$ ein Fluoratom oder ein Wasserstoffatom darstellt und $R^3$ ein Wasserstoffatom darstellt.

**9.** Therapeutisches Mittel zur Verwendung gemäß Anspruch 8, worin $R^1$ ein Wasserstoffatom darstellt, $R^2$ ein Fluoratom darstellt und $R^3$ ein Wasserstoffatom darstellt.

**10.** Therapeutisches Mittel zur Verwendung gemäß irgendeinem der Ansprüche 7 bis 9, welches ferner in Kombination mit einem oder mehr anderen therapeutischen Mitteln für eine RNA-Virusinfektion oder Wirkstoffen, die eine inhibierende Wirkung auf einen RNA-Virus zeigen, verwendet wird.

**11.** Therapeutisches Mittel zur Verwendung gemäß Anspruch 10, worin ein anderes therapeutisches Mittel für eine RNA-Virusinfektion oder ein anderer Wirkstoff, der eine inhibierende Wirkung auf einen RNA-Virus ausübt, Ribavirin ist.

**Revendications**

**1.** Agent thérapeutique pour l'utilisation dans le traitement d'une infection virale à ARN, qui comprend une combinaison d'un dérivé de pyrazine représenté par la formule [1] suivante ou un sel de celui-ci et un ou plusieurs types de composés choisis dans le groupe constitué d'un antifolate, d'un antimétabolite de thiopurine, d'une thiazofurine, d'un agent d'alkylation et d'un dérivé de xanthine :

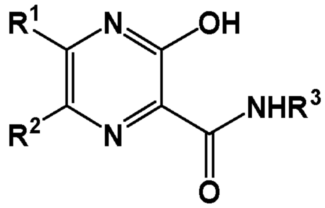

[1]

dans laquelle $R^1$ et $R^2$, qui sont identiques ou différents, représentent chacun un atome d'hydrogène ou un atome d'halogène ; et $R^3$ représente un atome d'hydrogène ou un groupe amino-protecteur, et dans lequel

l'antifolate est le méthotrexate ou le pralatrexate, l'antimétabolite thiopurine est un dérivé de la mercaptopurine représenté par la formule [2] suivante :

[Formule 2]

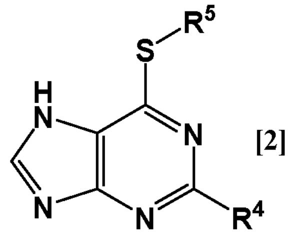

[2]

dans laquelle $R^4$ représente un atome d'hydrogène ou un groupe amino optionnellement protégé ; et $R^5$ représente un atome d'hydrogène ou la formule [3] suivante :

[Formule 3]

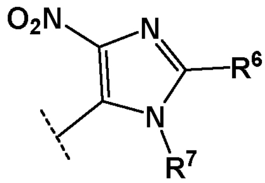

[3]

(dans laquelle $R^6$ représente un atome d'hydrogène, un groupe alkyle en $C_{1-6}$, ou un groupe carboxy ; et $R^7$ représente un atome d'hydrogène, un groupe alkyle en $C_{1-6}$, un groupe benzyle, ou un groupe p-nitrobenzyle), l'agent d'alkylation est le témozolomide, et le dérivé de xanthine est la théophylline.

**2.** L'agent thérapeutique pour l'utilisation selon la revendication 1, dans lequel $R^1$ représente un atome d'hydrogène, $R^2$ représente un atome de fluor ou un atome d'hydrogène, et $R^3$ représente un atome d'hydrogène.

**3.** L'agent thérapeutique pour l'utilisation selon la revendication 2, dans lequel $R^1$ représente un atome d'hydrogène, $R^2$ représente un atome de fluor, et $R^3$ représente un atome d'hydrogène.

**4.** L'agent thérapeutique pour l'utilisation selon la revendication 1, dans lequel l'antimétabolite thiopurine est la 6-mercaptopurine, l'azathioprine, ou la 6-thioguanine.

**5.** L'agent thérapeutique pour l'utilisation selon l'une quelconque des revendications 1 à 4, qui comprend en outre un ou plusieurs autres agents thérapeutiques pour les infections virales à ARN ou des médicaments qui exercent une action inhibitrice sur les virus à ARN.

**6.** L'agent thérapeutique pour l'utilisation selon la revendication 5, dans lequel un autre agent thérapeutique pour les infections virales à ARN ou un autre médicament qui exerce une action inhibitrice sur les virus à ARN est la ribavirine.

**7.** Agent thérapeutique pour l'utilisation dans le traitement d'une infection virale à ARN, comprenant un dérivé de

pyrazine représenté par la formule [1] suivante ou un sel de celui-ci, qui est utilisé en combinaison avec un ou plusieurs types de composés choisis dans le groupe constitué d'un antifolate, d'un antimétabolite de thiopurine, d'une thiazofurine, d'un agent d'alkylation et d'un dérivé de xanthine :

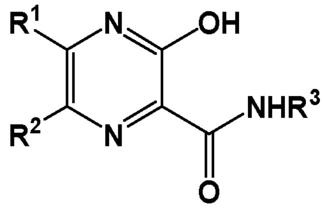
[1]

dans laquelle $R^1$ et $R^2$, qui sont identiques ou différents, représentent chacun un atome d'hydrogène ou un atome d'halogène ; et $R^3$ représente un atome d'hydrogène ou un groupe amino-protecteur, et dans lequel

l'antifolate est le méthotrexate ou le pralatrexate, l'antimétabolite thiopurine est un dérivé de la mercaptopurine représenté par la formule [2] suivante :

[Formule 2]

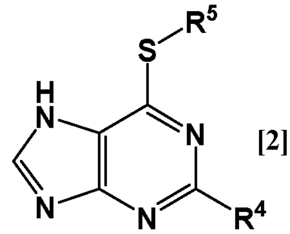
[2]

dans laquelle $R^4$ représente un atome d'hydrogène ou un groupe amino optionnellement protégé ; et $R^5$ représente un atome d'hydrogène ou la formule [3] suivante :

[Formule 3]

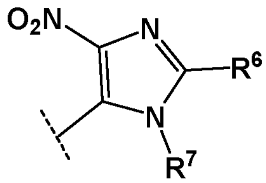
[3]

(dans laquelle $R^6$ représente un atome d'hydrogène, un groupe alkyle en $C_{1-6}$, ou un groupe carboxy ; et $R^7$ représente un atome d'hydrogène, un groupe alkyle en $C_{1-6}$, un groupe benzyle, ou un groupe p-nitrobenzyle), l'agent d'alkylation est le témozolomide, et le dérivé de xanthine est la théophylline.

**8.** L'agent thérapeutique pour l'utilisation selon la revendication 7, dans lequel $R^1$ représente un atome d'hydrogène, $R^2$ représente un atome de fluor ou un atome d'hydrogène, et $R^3$ représente un atome d'hydrogène.

**9.** L'agent thérapeutique pour l'utilisation selon la revendication 8, dans lequel $R^1$ représente un atome d'hydrogène, $R^2$ représente un atome de fluor, et $R^3$ représente un atome d'hydrogène.

**10.** L'agent thérapeutique pour l'utilisation selon l'une quelconque des revendications 7 à 9, qui est outre utilisé en combinaison avec un ou plusieurs autres agents thérapeutiques pour les infections virales à ARN ou des médicaments qui exercent une action inhibitrice sur les virus à ARN.

**11.** L'agent thérapeutique pour l'utilisation selon la revendication 10, dans lequel un autre agent thérapeutique pour les infections virales à ARN ou un autre médicament qui exerce une action inhibitrice sur les virus à ARN est la ribavirine.

[Fig. 1]

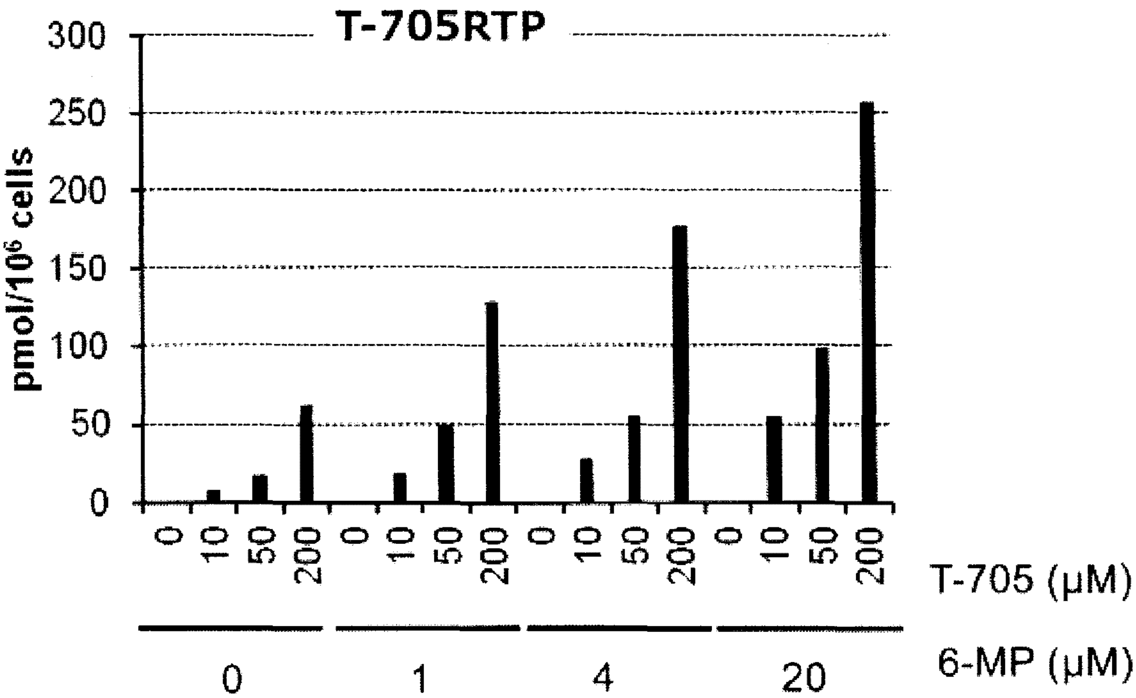
T-705RTP
pmol/10⁶ cells
300
250
200
150
100
50
0
0 10 50 200 0 10 50 200 0 10 50 200 0 10 50 200
T-705 (µM)
0 1 4 20
6-MP (µM)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008099874 A
- WO 2017202789 A
- WO 2011053812 A
- WO 0010569 A

### Non-patent literature cited in the description


- *Proc Jpn Acad Ser B Phys Biol Sci.*, vol. 93, 449-463
- *CDC Health Advisory:CDC issues interim recommendations for the use of influenza antivirals in the setting of oseltamivir Resistance among circulating influenza A (H1N1) viruses*, 2008
- **Y. FURUTA**. *Antiviral Research*, 2009, vol. 82, 95-102
- **K. THARMARAJAH et al.** *F1000Research*, 2017, vol. 6, 2114
- **J. R. STANGL et al.** *CLINICAL TRANSPLANTATION*, 2004, vol. 77, 562-567
- **Q. YANG**. *Medical Hypothesis*, 2004, vol. 62, 358-363
- **W. GREENE et al.** Protective Groups in Organic Synthesis. John Wiley & Sons, INC, 2014, 895-1193
- *Antimicrob Agents Chemother.*, 2005, vol. 49 (3), 981-6
- *Journal of Young Pharmacists*, 2019, vol. 11 (2), 117-121
- **NEUMANN, G. et al.** *J. Virol.*, 2000, vol. 74, 547-551
- **JONES, M. H. et al.** *Proc. Natl. Acad. Sci. USA*, 1988, vol. 85, 669-673
- **GRUMMT, I. et al.** *Cell*, 1986, vol. 45, 837-846
- **MATSUDA S** ; **KASAHARA T**. *Genes Environ*, 2018, vol. 40, 13
- *Antiviral Res.*, 2009, vol. 82 (3), 95-102
- *Mol Pharmacol.*, 2013, vol. 84 (4), 615-29